# EUROPEAN PATENT SPECIFICATION

(11) **EP 0 517 771 B1**
(45) Date of publication and mention of the grant of the patent: **14.04.1999**
(21) Application number: 91905164.9
(22) Date of filing: 01.03.1991
(51) Int. Cl.: A61K 9/22

(54) **DISPENSING DEVICE**
ABGABEVORRICHTUNG
MOYEN D'ADMINISTRATION MEDICAMENTEUSE

(30) Priority: 02.03.1990 GB 9004702
(43) Date of publication of application: 16.12.1992
(60) Divisional application: 98108892.5
(73) Proprietor: BTG INTERNATIONAL LIMITED, London EC4M 7SB (GB)
(72) Inventor: STEVENS, Howard Norman Ernest, Drymen, Stirlingshire G63 OBX (GB); RASHID, Abdul, Glasgow G41 3AV (GB); BAKHSHAEE, Massoud, Glasgow G64 2SN (GB)
(74) Representative: Dolan, Anthony Patrick
(86) International application number: GB9100317
(87) International publication number: WO9112795

(56) References cited:
- EP-A- 132 384
- EP-A- 384 646
- FR-A- 2 100 858
- GB-A- 436 236
- GB-A- 1 022 171
- GB-A- 2 206 046
- US-A- 4 898 733

## Description

This invention relates to novel devices for the controlled release of an active material into an aqueous environment, particularly the release of a pharmaceutically active drug into the human or the animal body. Specifically this invention relates to hollow devices which are designed to release their contents over a relatively short period following a controlled delay after administration.

A wide variety of devices for the controlled release of drugs have been proposed in the art. One of the simplest is the hard gelatin capsule containing an active material which is released very shortly after administration as the gelatin is rapidly dissolved. The devices which have been proposed in order to delay that release have been of a complex construction which has severely limited their utilisation.

We have now discovered a novel device which provides a controlled delay after administration after which the contents of the device are released. Such devices comprise a water impermeable capsule having at least one orifice wherein said orifice is closed by the insertion of a precompressed plug which is soluble or erodible in water. Such devices may be constructed simply and economically and enable the pulsed release to be achieved in a controlled and reproducible manner.

Accordingly, from one aspect this invention provides a pulsed release device for releasing the contents of a capsule into an aqueous medium which comprises a water impermeable capsule having at least one orifice wherein the orifice is closed with a water soluble or water erodible precompressed plug adapted so as to close said orifice.

The water soluble or erodible precompressed plug is preferably designed so as to provide a controlled delay between the introduction of the device into an aqueous environment and the exposure of the contents of the cavity to that environment.

The devices of this invention are preferably constructed so that the area of their exterior surface which is soluble or dispersable comprises only a minor proportion of the total area of the exterior surface of the device. Generally no more than 40% of and preferably less than 20% of the surface area will be formed from water soluble or dispersable materials.

The areas of the water soluble or dispersable material will normally be sufficient to allow the rapid ingress of water into the cavity when the material has dissolved or dispersed, say at least 5% and more preferably at least 10% of the surface area. However, it may constitute a smaller percentage and in some devices such a feature may be preferred. Devices wherein the area of the water soluble material comprises less than 5% and even less than 1% of the total exterior surface area of the device are within the scope of this invention.

The water impermeable portion of the walls may be formed from a variety of materials. They may be of homogeneous construction or they may be laminated. The materials used to form the walls may be impermeable but permeable materials may be utilised provided that the overall construction does not permit the ingress of significant quantities of water prior to the dissolution or dispersion of the soluble portion of the wall. Examples of impermeable materials which may be utilised include polyethylene, polypropylene, poly(methylmethacrylate) polyvinyl chloride, polystyrene, polyurethanes, polytetrafluoroethylene, nylons, polyformaldehydes, cellulose acetate and nitrocellulose.

In the case of oral dosage forms for the administration of pharmaceutically active ingredients the materials used in the construction of the device should be biologically and medically compatible with; non-allergenic and insoluble in and non-irritating to body fluids and tissues. They may be formed from such a material which is biodegradable.

The water soluble or dispersable material is preferably one which can readily be formed into the configuration required for a particular device. Thus materials or blends of materials which are amenable to forming by direct powder compression or by wet granulation and compression or other such techniques are preferred for use in this invention. Those materials known to be useful as excipients in pharmaceutical tablets are generally suitable for use in the formation of the plugs useful in the devices of this invention. Examples of such materials include sugars such as lactose, sucrose, dextrose, sorbitol and mannitol; dextrins; polycarboxylic acids such as ascorbic acid, malic acid, fumaric acid, citric acid and tartaric acid; polyethylene glycols, polyvinyl pyrrolidone, and polyvinylacetate. All of these materials can be compressed to form suitable plugs.

A further group of materials which may be utilised is those which can be processed into a form which erodes when exposed to an aqueous environment. Examples of materials which may be utilised include inorganic salts such as calcium and magnesium carbonates, calcium phosphate, calcium sulphate, cellulose compounds and derivatives such as microcrystalline cellulose, microfine cellulose and ethyl cellulose, hydrolysed starches, waxes, glycerides and hydrogenated vegetable oils. They may be processed into a erodible form by the incorporation of a water soluble material (including materials such as those listed above) or by the incorporation of a disintegrant or by a combination of these two approaches. Materials which are useful as disintegrants include all those known in the art of pharmaceutical technology especially starch, carboxymethylstarch, cross-linked PVP and alginic acid and including those sold under the proprietory names Polyplasdone and Acdisol.

The delay in the release of the contents of the cavity varies with the nature of the material used to form the precompressed plug and with the manner in which that plug is formed. The actual length of delay provided by a particular plug in a particular device in a controlled environment may be determined by routine experiment. It is within the knowledge of the art to vary the formulation and production of the plugs so as to produce the desired time delay.

These soluble or erodible materials either separately or a combination may be formed into suitable configurations using classical pharmaceutical techniques. They may further comprise effervescent materials. They may be mixed with pH-sensitive materials such as cellulose acetate phthalate, hydroxypropylmethylcellulose phthalate, polyvinylacetate phthalate and polymethacrylate derivatives. They may comprise an active material which is released into the surrounding environment during the period of dissolution or dispersion prior to the release of the contents of the interior of the capsule.

The devices of this invention may be formed in any convenient shape, for example spheroidal, ellipsoidal or cylindrical. Capsules which are generally cylindrical are preferred. A preferred form of a device according to this invention comprises a hollow cylinder open at one or both ends having a water impermeable construction, said device having a precompressed plug of water soluble or erodible material inserted so as to close the open end or ends. Such devices may be readily formed, e.g. from an extruded plastic tube cut into lengths and optionally sealed at one end.

Alternatively the capsule may be formed by forming a cylinder around a rod; by coating a solution of polymer or organosol onto a former; by compression or injection moulding of a suitable thermoplastic polymer; by powder compression or by direct reaction moulding.

The cylindrical devices are conveniently of a size which may be swallowed and hence they find use as oral dosage forms for man in particular, but also in animals. Typically the length of the hollow cylinder will be in the range 5 to 100 mm, preferably 10 to 30 mm and the external diameter will be in the range 1 mm to 20 mm. Typically the devices will have external dimensions corresponding to those of known oral dosage forms e.g. capsules having sizes in the range triple zero to zero and from 1 to 8. In another embodiment the devices of this invention may be significantly smaller so as to facilitate the inclusion of a plurality of devices in a single dosage form, e.g. a capsule. This enables different release patterns to be obtained.

The orifice in the wall of the capsule may adopt any convenient configuration but in the preferred case will be a circular in cross-section and uniform throughout its depth. Such orifices may readily be closed by the insertion of a right cylindrical plug. The delay in the release of the contents of the capsule is dependant to the depth of any particular precompressed plug. The device is constructed so as to ensure that the depth of the walls of the orifice is at least equal to the depth of the plug which is required to be accommodated. In the preferred embodiment the plugs may have a depth of from 0.5 to 10.0 mm more preferably 1.0 to 5.0 mm. The plug will preferably be positioned so that its exposed surface is flush with the exterior wall of the capsule.

The contents of the device may take the form of the active material as such, e.g. as a particulate solid or may take the form of any other convenient dosage form. For example, the active material may be combined with a conventional excipient and be introduced into the device as a powder or as a fluid solution or suspension (provided that the fluid medium does not interact significantly with the materials used to form the walls of the device) or take the form of compressed tablets of excipient and carrier. Either a single tablet or a plurality of such tablets may be introduced. A further alternative is to introduce the active material in a modified dosage form, e.g. a coated material such as is described in British Patent 2112730. This enables the release profile of the device to be modified, e.g. where a crystalline active material is employed it will be released as a single sharp pulse of active material, whereas where a modified dosage form is employed that form will be released into the environment after the pre-determined delay and the subsequent release profile will be that of the modified dosage form. In another preferred embodiment the devices of this invention may provide a multiple pulse release. Such devices comprise a plurality of hollow compartments each separated from the other by a soluble or dispersable wall and arranged so that the contents of the outermost compartment are released before the wall of the next compartment is exposed to the aqueous environment. An example of such a device is a hollow capsule containing alternate layers of active material and precompressed plugs formed from soluble or erodible materials. Any combination of these alternatives may be employed.

The devices of this invention find wide application in medical, including veterinary, contexts and in horticulture and agriculture as well as outside these areas.

Specific classes of drug which may be utilised as the active material in a pulsed release device of this invention include hypnotics, sedatives, tranquilisers, antipyretics, anti-inflammatory agents, anti-histamines, anti-tussives, anti-convulsants, anti-asthmatics, muscle relaxants, anti-tumour agents, for example those for the treatment of malignant neoplasia, local anaesthetics, anti-Parkinson agents, topical or dermatological agents, diuretics, for example those containing potassium, such as potassium iodide, preparations for the treatment of mental illness, for example preparations containing lithium for use in the treatment of manic depression or containing prostaglandins for the treatment of schizophrenia, anti-spasmodics, anti-ulcer agents, β blockers such as atenolol and metoprolol; calcium antagonists such as nifedipine and nitrendipine, ACE inhibitors such as enalapril and captopril, β₂ agonists such as salbutamol and terbutaline, preparations containing various substances for the treatment of infection by pathogens including antifungal agents, for example metronidazole, anti-parasitic agents and other anti-microbials, anti-malarials, cardiovascular agents, preparations containing hormones, for example androgenic, estrogenic and progestational hormones, notably steroids such as oestradiol, sympathomimetic agents, hypoglycaemic agents, contraceptives, nutritional agents, peptides and proteins, nitrates such as isorbide dinitrate, mononitrate and GTN; xanthines such as theophylline; NSAID's such as piroxicam and diclofenac; benzodiazepines such as triazolam and zopiclone; α blockers such as prazosine and alfuzosine; antivirals such as acyclovir, zidovudine and ampligen, cephalosporins such as cefaclor, antispasmodics such as alverine and salicylates such as 5 amino salicylic acid; preparations containing enzymes of various types of activity, for example chymotrypsin, preparations containing analgesics, for example aspirin, andagents with many other types of action including nematocides and other agents of veterinary application. Mixtures of active substances may be incorporated into the controlled release device.

The controlled release devices of this invention are also useful in the treatment of diabetes and pernicious anaemia where, for example, the controlled release of insulin and cobalamin, respectively, may be utilised.

Moreover, the release devices of this invention are suited to treatment, both prophylactic and therapeutic, of tropical diseases; for example malaria, leprosy, schistosomiasis and clonorchiasis. Examples of drugs which can be used as biologically active substance in release devices of this invention for the treatment of these and other tropical diseases include quinine, sulphonamides, rifamycin, clofazimine, thiambutasine, chlorphenyl derivatives, chlorguamide, cycloguanil, pyrimethamine, sulphadiazine, trimethoprim, quinoline derivatives such as pamaquine, chloroquine, pentaquine, primaquine and amodiquine, pararosaniline, sulphamethizole, quinacrine, dapsone, sodium sulphoxone, sulphetrone, sodium hydnocarpate and sodium chaulmoograte. Drugs of particular effectiveness are cycloguanil, pyrimethamine and sulphadiazine.

The release devices of this invention are also very well suited to veterinary applications. Examples include preparations of antibiotics for general anti bacterial activity and also in the treatment of anaplasmosis in cattle; preparations for provision of a wide spectrum of activity against both ectoparasites, for example termites and endoparasites including anthropods, arrested larvae stages of nematodes, lungworms and general strongyles: these may comprise avermectins; preparations for provision of activity against tremotode, cestode and roundworm infections: these may comprise amoscanate and praziquantel: preparations for provision of activity against theileria in cattle: these may comprise biologically active naphthoquinones such as menoctone; preparations for provision of activity against babesiosis in cattle, horses and dogs: these may comprise berenil, amidocarb and diampron; preparation for provision of activity against liver fluke in sheep and cattle and against Haemonchus species: these may comprise closantel.

The devices of the present invention may also be combined with another dosage form which will combine the release profile of the novel devices with that of the other dosage form. Thus, for example, two separate devices according to this invention may be joined end to end so that the active materials are separated by a wall or precompressed plug. Alternatively a device according to this invention may be joined to any other controlled release device (of appropriate dimensions).

A further preferred embodiment of this invention comprises devices coated with an enteric coating so as to pass through the stomach and release the active material in the intestine. Such devices can be designed so as to release the active a set period after they pass out of the stomach. They may be designed to release in the colon. Any conventional enteric coating agent may be employed for example cellulose acetate phthalate, polyvinyl acetate phthalate, hydroxypropylmethyl cellulose phthalate and polymethacrylate derivatives.

A preferred construction utilises an impermeable coating to cover the exterior of a capsule formed from a water soluble material. The coating may conveniently be formed by dipping a capsule in a solution of any of the above-mentioned materials so as to form a layer which is impermeable to water. The capsule may also be spray coated with solutions of the above materials in which use the exterior has an impermeable coating and the interior may be partially coated. A preferred class of capsules are the conventional hard gelatin capsules coated with a polyvinyl chloride or a polyvinyl chloride acetate copolymer or ethyl cellulose solution. Such devices are advantageous in that they are simple to construct and insofar as their soluble interiors dissolve in the aqueous medium leaving the thin flexible coating to be eliminated from the body.

In use the devices of the invention may be modified so as to resemble known capusles. In particular the cylindrical devices of the preferred embodiments may be combined with a half capsule formed of e.g. hard gelation to form such a device.

The invention is illustrated by the following Examples:

### Example 1

Two capsules were produced from hollow cylinders of flexible PVC closed at one end. The capsules had a length of 17 mm and an internal diameter of 6.3 mm. A charge of particulate salbutamol sulphate was placed in the interior of each capsule. The open ends were closed with a tablet formed by compression of Eudragit L100-55 (a proprietory brand of a methacrylic acid - ethyl acrylate copolymer and sold by the Rohm-Pharma company). In the first instance 56 mg of Eudragit was compressed in a die of diameter 7.18 mm to form a tablet having a thickness of 1.27mm. In the second instance 47 mg of Eudragit was compressed in the same die to form a tablet having a thickness of 1.07 mm.

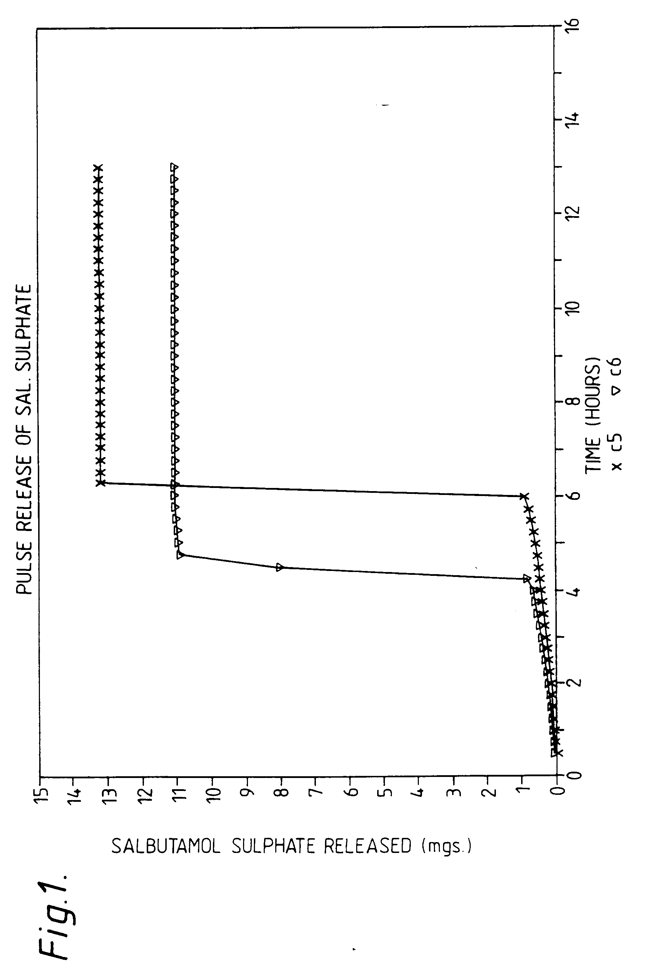

The release profile of these devices in water was measured using a US Pharmacopeia dissolution bath in which the aqueous medium was a buffer solution having a pH of 7.6 The results are shown in Figure 1 in which the results for the first device are indicated as X and for the second as ∇.

### Example 2

Two multiple pulse release devices were prepared using a capsule formed from flexible PVC identical to that used in Example 1 which was filled with alternate layers of salbutamol sulphate and compressed tablets made from Eudragit L100-55 using the quantities shown in the following table (listing the compositions in ascending order starting with the layer placed at the bottom, the closed end, of the capsule).

**Table 1**

| | Device 1 | Device 2 |
|---|---|---|
| Salbutamol | 10.1 mg | 9.5 mg |
| Eudragit | 48.8 mg | 49.3 mg |
| Salbutamol | 9.7 mg | 9.9 mg |
| Eudragit | 49.7 mg | 50.3 mg |
| Salbutamol | 9.3 mg | 10.2 mg |
| Eudragit | 49.1 mg | 50.0 mg |
| Salbutamol | 9.4 mg | 9.8 mg |
| Eudragit | 48.5 mg | 51.1 mg |

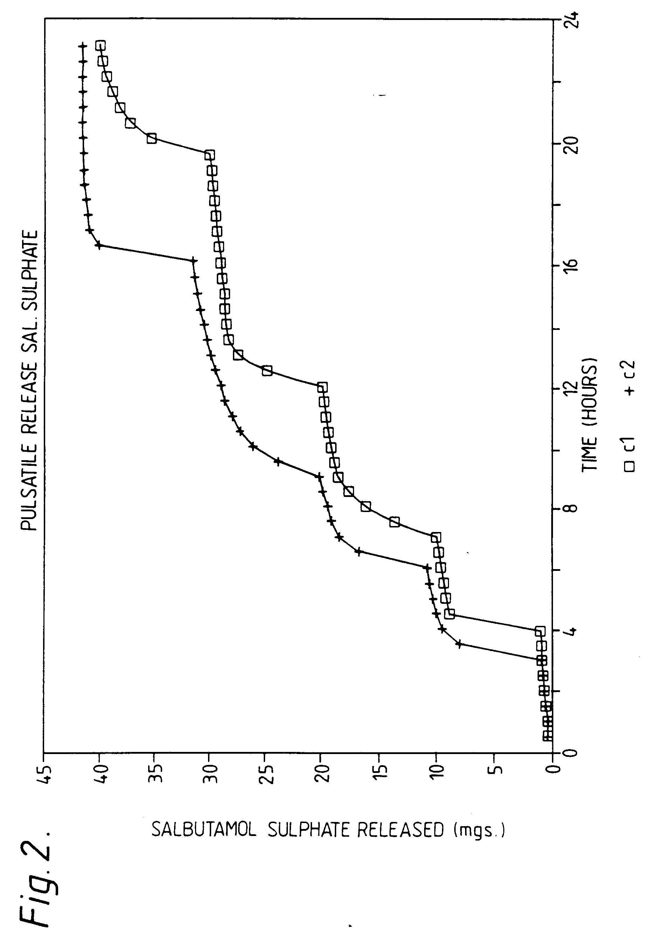

The release profile was tested using the same procedure as in Example 1. The results are shown in Figure 2 (the first device indicated as □ and the second as +).

### Example 3

Three pulsed release devices were assembled using a capsule made from low density polyethylene (LDPE) having an overall length of 18mm, an internal diameter of 6.33mm and a wall thickness of 20 thousandth of an inch which were filled with sodium chloride and salbutamol sulphate placed such that the drug was positioned just below the soluble plug. The soluble plug was prepared by compacting a mixture of lactose (98.5 wt %), Aerosil 200 (1 wt %) and Magnesium Stearate (0.5 wt %) on Manesty Type F3 Tabletting Machine. Details are shown in Table 2 below.

**Table 2**

| | Device Number | | |
|---|---|---|---|
| | 4 | 5 | 6 |
| Salbutamol (mg) | 9.2 | 9.1 | 10.1 |
| Sodium Chloride (mg) | 504.8 | 516.3 | 514.9 |

| Soluble Plug | | | |
|---|---|---|---|
| Diameter (mm) | 6.4 | 6.4 | 6.4 |
| Thickness (mm) | 2.81 | 2.81 | 2.81 |
| Weight (mg) | 120 | 120 | 120 |

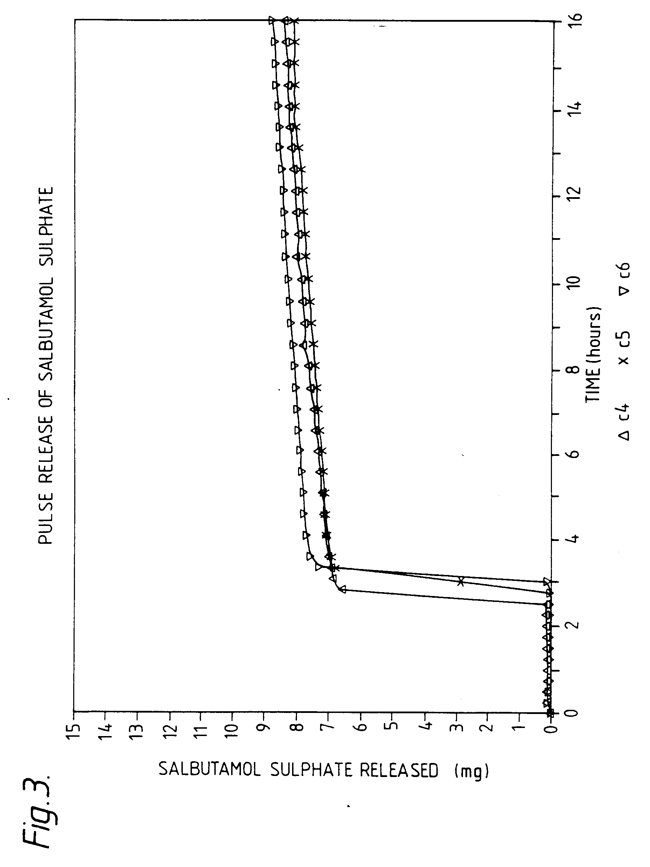

The release profile was determined in water using the same procedure as described in Example 1. The results are graphically shown in Figure 3.

### Example 4

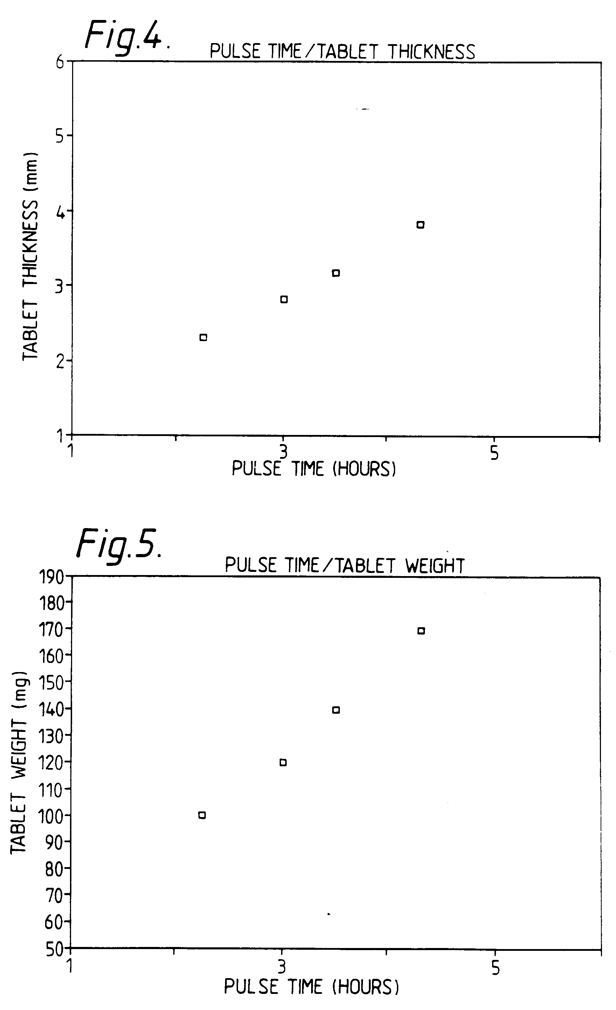

Several devices were assembled by utilising the same components (i.e. same capsule, same plug formulation) as in Example 3 but the thickness and weight of the plug was varied. The devices were tested as per Example 3 and the mean results are shown in Figures 4, 5 and in Table 3 below.

**Table 3**

| MEAN TABLET WT (mg) | MEAN THICKNESS (mm) | MEAN PULSE TIME (h) |
|---|---|---|
| 100 | 2.3 | 2.25 |
| 120 | 2.81 | 3.00 |
| 140 | 3.16 | 3.50 |
| 170 | 3.82 | 4.30 |

### Example 5

Four devices were prepared by utilising the capsule identical to that used in Example 4. The soluble plugs were prepared by compacting a mixture of lactose (70 wt %), Polyvinyl pyrrolidone (m wt 700,000) (20 wt %) and Magnesium Stearate (1 wt %) on Manesty F3 type tabletting machine. The details are shown in Table 4 below.

**Table 4**

| | Device Number | | | |
|---|---|---|---|---|
| | 1 | 2 | 3 | 4 |
| Sodium Chloride (mg) | 505.2 | 504.7 | 515.0 | 513.0 |
| Salbutamol sulphate (mg) | 10.4 | 10.2 | 9.7 | 10.5 |

| Soluble Plug | | | | |
|---|---|---|---|---|
| Diameter (mm) | 6.4 | 6.4 | 6.4 | 6.4 |
| Thickness (mm) | 3.06 | 3.10 | 4.26 | 4.23 |
| Weight (mg) | 118.7 | 120.5 | 169.1 | 167.6 |

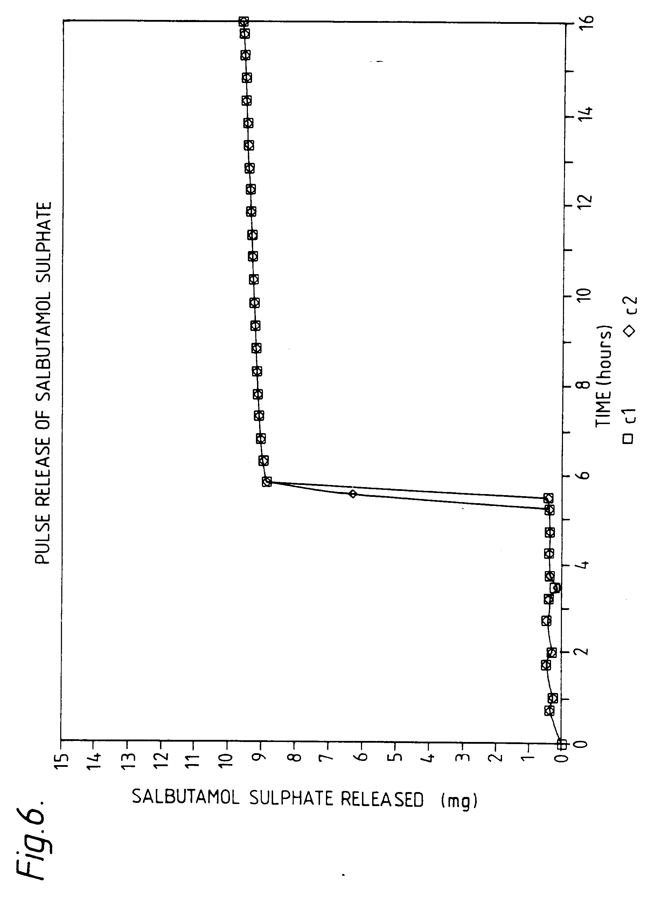

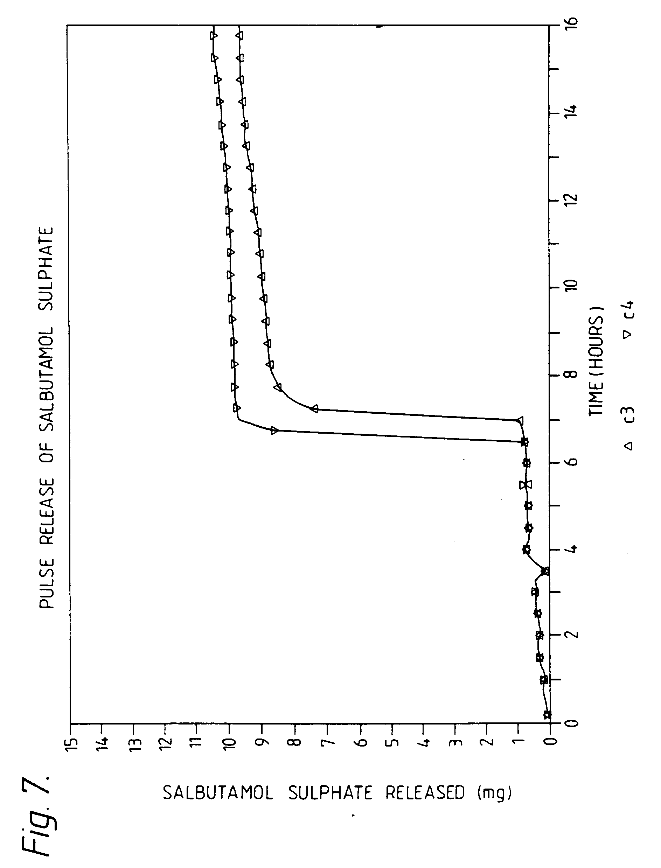

The release profiles were determined using the same procedure as described in Example 3. The results are shown in Figures 6 and 7.

### Example 6

Six devices were prepared by utilising the capsule described in Example 5. The plug was prepared by compacting a mixture of lactose (79 wt %), Polyethylene glycol Mn 8000 (20 wt %) and Magnesium Stearate (1 wt %) on a Manesty F3 tabletting machine. Details are shown in Table 5 below.

**Table 5**

| | Device Number | | | | | |
|---|---|---|---|---|---|---|
| | 1 □ | 2 + | 3 ◇ | 4 △ | 5 X | 6 ∇ |
| Sodium Chloride (mg) | 610.4 | 609.7 | 609.4 | 545.6 | 545.6 | 545.5 |
| Salbutamol sulphate (mg) | 9.6 | 9.4 | 9.9 | 9.5 | 10.3 | 10.2 |

| Soluble Plug | | | | | | |
|---|---|---|---|---|---|---|
| Diameter (mm) | 6.4 | 6.4 | 6.4 | 6.4 | 6.4 | 6.4 |
| Thickness (mm) | 2.2 | 2.2 | 2.2 | 3.8 | 3.8 | 3.8 |
| Weight (mg) | 98.0 | 98.0 | 98.0 | 166.5 | 166.5 | 166.5 |

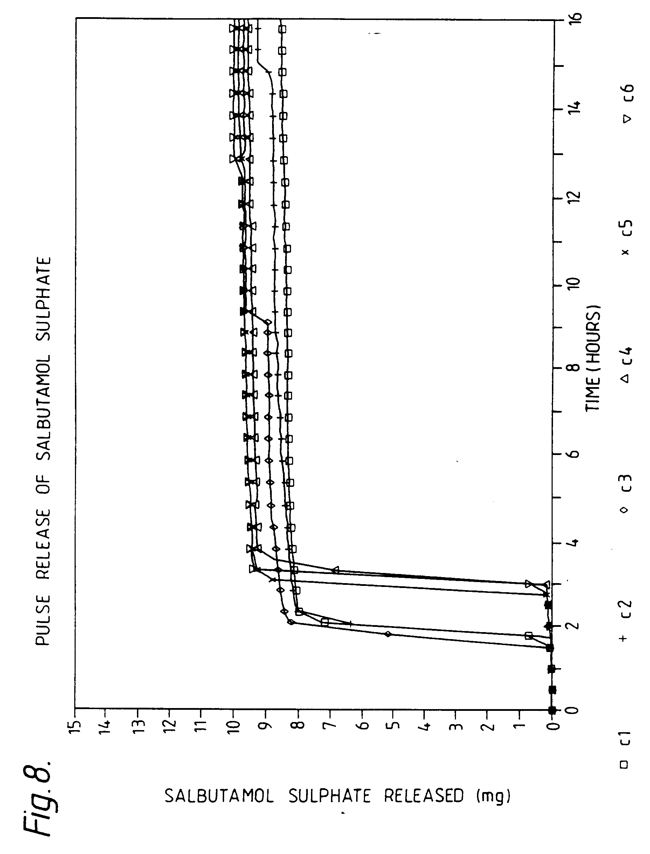

The release profile was determined as in Example 3. The results are shown graphically in Figure 8.

### Example 7

A series of devices were prepared utilising the capsule described in Example 3. A series of tablets were prepared using the following excepients.
Lactose (LAC)
Aerosil 2000 (AER)
PVP (Polyvinylpyrolidene M.W. 700,000) - (PVP)
CUTINA HR (CUT)*
Magnesium steurate (Mg St)

* a hydrogenated vegetable oil sold by the Henkel Company.

The appropriate quantities of the excipients were weighed in a glass jar. The jar was secured with a lid and the contents mixed using a Turbula mixer for the time specified in the Table. The mixture was then compressed into tablets of various weights using a Manesty F3 tabletting machine. The open end of the capsule was closed by placing the tablet in the flush position and the dissolution time determined in the manner described in Example 1. The results are shown in the following table.

**TABLE 1**

| Tablet Formation | Ratio of Components (wt %) | Mixing Time (min) | Average Tablet Weight (mg) | Average Tablet Thickness (mm) | Average Pulse Time (hr) |
|---|---|---|---|---|---|
| LAC/AER/MG St | 98.5/1/0.5 | 15 | 140 | 3.16 | 3.43 |
| " | 98.5/1/0.5 | 15 | 170 | 3.82 | 4.30 |
| LAC/PVP/MG St | 79/20/1 | 15 | 150.1 | 3.49 | 4.40 |
| LAC/CUT/Mg St | 96/3/1 | 15 | 119.0 | 2.66 | 5.45 |
| " | 98/1/1 | 20 | 145.3 | 3.21 | 7.52 |
| LAC/Mg St | 98/2 | 15 | 170.4 | 3.74 | 9.04 |
| " | 98/2 | 15 | 120.2 | 2.62 | 5.00 |
| " | 98.25/1/75 | 30 | 169.7 | 3.75 | 8.34 |
| " | 98.25/1/75 | 60 | 171.2 | 3.78 | 9.14 |
| " | 98/2 | 60 | 169.5 | 3.80 | 9.56 |
| " | 98/2 | 60 | 145.2 | 3.20 | 7.88 |
| " | 98/2 | 60 | 120.3 | 2.65 | 5.96 |
| LAC/Mg St (MULTIPULSE) | 98.5/1.5 | 60 | 121.5 | 2.65 | 4.46 |
| | | | 120.5 | 2.63 | 11.58 |
| | | | 120.7 | 2.63 | 21.71 |
| | | | 120.8 | 2.63 | 31.92 |

### Example 8

A series of devices were made up in the manner described in Example 7 using the following excipients:
CUTINA (CUT)
EMCOSOY (EMCO)*
EXPLOTAB (EXPLO)+

* a soya polysaccharide excipient sold by Forum Chemicals Ltd.
+ a sodium starch glycollate disintegrant sold by Forum Chemicals Ltd.

The compositions of the tablets are the results obtained are summarised in Table 2.

**TABLE 2**

| Tablet Formation | Ratio of Components (wt %) | Mixing Time (min) | Average Tablet Weight (mg) | Average Tablet Thickness (mm) | Average Pulse Time (hr) |
|---|---|---|---|---|---|
| CUT/EMCO.LAC | 70:15:15 | 25 | 114.3 | 3.65 | 6.42 |
| CUT/EXPLO | 80:20 | 25 | 125.2 | 3.69 | 7.33 |
| Mg St added as 0.5% of total weight in both cases | | | | | |

## Claims

1. A pulsed release capsule suitable for human oral administration having a water-impermeable external surface and an orifice closed by a plug through which orifice the capsule contents are to be released in the human body characterised in that the capsule and plug together form a cavity and the orifice is plugged with a water-soluble or water erodible precompressed plug composed of a pharmaceutically acceptable material which dissolves or erodes after a time in the human body to allow pulsed release of the capsule contents wherein the area of water soluble or erodible material is sufficient to allow the rapid ingress of water into the cavity when the material has dissolved or eroded.

2. A capsule according to Claim 1 characterised in that it is the form of a hollow cylinder having an orifice at one or both ends.

3. A capsule according to any one of the preceding claims characterised in that it has a length of from 10 to 30 mm.

4. A capsule according to any one of the preceding claims characterised in that the exposed area of the plug comprises no more than 40% of the total exterior surface area of the capsule.

5. A capsule according to any of the preceding claims in which the exposed surface of the plug is flush with the end of the capsule.

6. A capsule according to any of the preceding claims in which the plug has a depth of from 0.5 to 10.0 mm.

7. A device according to Claim 6, in which the plug has a depth of from 1.0 to 5.0 rnm.

8. A device according to any of the preceding claims, in which the carrier material is a sugar.

9. A device according to any of the preceding claims, in which the carrier material is a cellulose compound or derivative.

10. A capsule according to any of the preceding claims, characterised in that the plug is formed by compression of the pharmaceutically acceptable material.

11. A capsule according to any of the preceding claims, formed from a water impermeable material selected from polyethylene, polypropylene, poly (methylmethacrylate), polyvinyl chloride, polystyrene, polyurethanes, polytetrafluoroethylene, nylons, polyformaldehydes, polyesters, cellulose acetate and nitro cellulose.

12. A capsule according to Claim 11, in which the water impermeable material is a thermoplastic.

13. A capsule according to Claim 11 or 12, in which the water impermeable material is low density polyethylene.

14. A capsule according to any of Claim 1 to 12, in which the capsule is formed from a water soluble material coated with a water impermeable material.

15. A capsule according to Claim 14, in which the water soluble material is gelatin or starch.

16. A capsule according to Claim 14 or 15 which the impermeable coating is polyvinyl chloride.

17. A capsule according to any of the preceding claims, containing a pharmaceutically active compound.

18. A capsule according to Claim 17, in which the active material is a particulate solid.

19. A capsule according to Claim 18, in which the active compound is present as a modified dosage form having a release profile other than that of a single pulse.

20. A capsule according to any of the preceding claims, having an external surface provided with an enteric coating.

21. A method of preparation of a pulsed release capsule comprising the steps of inserting into the orifice of a capsule having a water impermeable surface, a precompressed water soluble or erodible plug composed of a pharmaceutically acceptible material which dissolves or erodes in water after a time in the human body, the plug and the capsule together defining a cavity containing the contents of the capsule wherein the area of the water soluble or erodible material is sufficient to allow the rapid ingress of water into the cavity when the material has dissolved or eroded.

## Patentansprüche

1. Kapsel für eine gepulste Freisetzung, die zur oralen Verabreichung an Menschen geeignet ist, mit einer wasserundurchlässigen äußeren Oberfläche und einer Öffnung, die durch einen Stopfen verschlossen ist, wobei durch die Öffnung der Inhalt der Kapsel in dem menschlichen Körper freigesetzt werden kann, dadurch gekennzeichnet, daß die Kapsel und der Stopfen zusammen einen Hohlraum bilden und die Öffnung verschlossen ist mit einem in Wasser löslichen oder durch Wasser erodierbaren vorverdichteten Stopfen, gebildet aus einem pharmazeutisch annehmbaren Material, das sich nach einer Zeit in dem menschlichen Körper löst oder erodiert wird, um eine gepulste Freisetzung des Kapselinhalts zu ermöglichen, wobei die Fläche des in Wasser löslichen oder erodierbaren Materials ausreichend ist, um den schnellen Eintritt von Wasser in den Hohlraum zu ermöglichen, wenn das Material gelöst oder erodiert worden ist.

2. Kapsel nach Anspruch 1, dadurch gekennzeichnet, daß sie die Form eines Hohlzylinders mit einer Öffnung an einem oder beiden Enden hat.

3. Kapsel nach einem der vorangehenden Ansprüche, dadurch gekennzeichnet, daß sie eine Länge von 10 bis 30 mm hat.

4. Kapsel nach einem der vorangehenden Ansprüche, dadurch gekennzeichnet, daß die freiliegende Fläche des Stopfens nicht mehr als 40 % der gesamten äußeren Oberfläche der Kapsel ausmacht.

5. Kapsel nach einem der vorangehenden Ansprüche, wobei die freiliegende Oberfläche des Stopfens mit dem Ende der Kapsel in einer Ebene liegt.

6. Kapsel nach einem der vorangehenden Ansprüche, wobei der Stopfen eine Tiefe von 0,5 bis 10,0 mm hat.

7. Vorrichtung nach Anspruch 6, wobei der Stopfen eine Tiefe von 1,0 bis 5,0 mm hat.

8. Vorrichtung nach einem der vorangehenden Ansprüche, wobei das Trägermaterial ein Zucker ist.

9. Vorrichtung nach einem der vorangehenden Ansprüche, wobei das Trägermaterial eine Celluloseverbindung oder ein Cellulosederivat ist.

10. Kapsel nach einem der vorangehenden Ansprüche, dadurch gekennzeichnet, daß der Stopfen durch Preßverdichten des pharmazeutisch annehmbaren Materials gebildet ist.

11. Kapsel nach einem der vorangehenden Ansprüche, gebildet aus einem wasserundurchlässigen Material, ausgewählt aus Polyethylen, Polypropylen, Poly(methylmethacrylat), Polyvinylchlorid, Polystyrol, Polyurethanen, Polytetrafluorethylen, Nylonarten, Polyformaldehyden, Polyestern, Celluloseacetat und Nitrocellulose.

12. Kapsel nach Anspruch 11, wobei das wasserundurchlässige Material ein thermoplastisches Material ist.

13. Kapsel nach Anspruch 11 oder 12, wobei das wasserundurchlässige Material Polyethylen niederer Dichte ist.

14. Kapsel nach einem der Ansprüche 1 bis 12, wobei die Kapsel aus einem wasserlöslichen Material gebildet ist, das mit einem wasserundurchlässigen Material überzogen ist.

15. Kapsel nach Anspruch 14, wobei das wasserlösliche Material Gelatine oder Stärke ist.

16. Kapsel nach Anspruch 14 oder 15, wobei der undurchlässige Überzug Polyvinylchlorid ist.

17. Kapsel nach einem der vorangehenden Ansprüche, enthaltend eine pharmazeutisch wirksame Verbindung.

18. Kapsel nach Anspruch 17, wobei der Wirkstoff ein teilchenförmiger Feststoff ist.

19. Kapsel nach Anspruch 18, wobei der Wirkstoff als modifizierte Dosierungsform mit einem Freisetzungsprofil, das anders ist als das eines einzelnen Pulses, vorliegt.

20. Kapsel nach einem der vorangehenden Ansprüche, die eine äußere Oberfläche aufweist, die mit einem enterischen oder darmlöslichen Überzug versehen ist.

21. Verfahren zur Herstellung einer Kapsel für eine gepulste Freisetzung, umfassend die Stufen eines Einsetzens eines vorverdichteten in Wasser löslichen oder erodierbaren Stopfens, gebildet aus einem pharmazeutisch annehmbaren Material, das sich nach einer Zeit im menschlichen Körper in Wasser löst oder erodiert wird, in die Öffnung einer Kapsel mit einer wasserundurchlässigen Oberfläche, wobei der Stopfen und die Kapsel zusammen einen Hohlraum bilden, der den Inhalt der Kapsel enthält, wobei die Fläche des wasserlöslichen oder erodierbaren Materials ausreicht, um den schnellen Eintritt von Wasser in den Hohlraum zu ermöglichen, wenn das Material gelöst oder erodiert worden ist.

## Revendications

1. Capsule à libération contrôlée par impulsions appropriée pour l'administration orale à l'humain, ayant une surface externe imperméable à l'eau et un orifice fermé par un bouchon, orifice au travers duquel le contenu de la capsule doit être libéré dans le corps humain, caractérisée en ce que la capsule et le bouchon forment ensemble une cavité et l'orifice est bouché avec un bouchon précomprimé hydrosoluble ou érodable par l'eau composé d'une matière pharmaceutiquement acceptable qui se dissout ou s'érode après un certain temps dans le corps humain pour permettre une libération contrôlée par impulsions du contenu de la capsule, dans laquelle l'aire de la matière hydrosoluble ou érodable par l'eau est suffisante pour permettre la pénétration rapide d'eau dans la cavité lorsque la matière s'est dissoute ou érodée.

2. Capsule selon la revendication 1, caractérisée en ce qu'elle a la forme d'un cylindre creux ayant un orifice à l'une des extrémités ou aux deux.

3. Capsule selon l'une quelconque des revendications précédentes, caractérisée en ce qu'elle a une longueur de 10 à 30 mm.

4. Capsule selon l'une quelconque des revendications précédentes, caractérisée en ce que l'aire du bouchon exposée ne représente pas plus de 40 % de l'aire de surface extérieure totale de la capsule.

5. Capsule selon l'une quelconque des revendications précédentes, dans laquelle la surface exposée du bouchon est de niveau avec l'extrémité de la capsule.

6. Capsule selon l'une quelconque des revendications précédentes, dans laquelle le bouchon a une profondeur de 0,5 à 10,0 mm.

7. Dispositif selon la revendication 6, dans lequel le bouchon a une profondeur de 1,0 à 5,0 mm.

8. Dispositif selon l'une quelconque des revendications précédentes, dans lequel la matière véhicule est un sucre.

9. Dispositif selon l'une quelconque des revendications précédentes, dans lequel la matière véhicule est un composé ou dérivé de cellulose.

10. Capsule selon l'une quelconque des revendications précédentes, caractérisée en ce que le bouchon est formé en comprimant la matière pharmaceutiquement acceptable.

11. Capsule selon l'une quelconque des revendications précédentes, formée à partir d'une matière imperméable à l'eau choisie parmi un polyéthylène, un polypropylène, un poly(méthacrylate de méthyle), un chlorure de polyvinyle, un polystyrène, les polyuréthannes, un polytétrafluoroéthylène, les nylons, les polyformaldéhydes, les polyesters, l'acétate de cellulose et la nitrocellulose.

12. Capsule selon la revendication 11, dans laquelle la matière imperméable à l'eau est une matière thermoplastique.

13. Capsule selon la revendication 11 ou 12, dans laquelle la matière imperméable à l'eau est un polyéthylène basse densité.

14. Capsule selon l'une quelconque des revendications 1 à 12, la capsule étant formée à partir d'une matière hydrosoluble revêtue avec une matière imperméable à l'eau.

15. Capsule selon la revendication 14, dans laquelle la matière hydrosoluble est la gélatine ou l'amidon.

16. Capsule selon la revendication 14 ou 15, dans laquelle le revêtement imperméable est un chlorure de polvvinyle.

17. Capsule selon l'une quelconque des revendications précédentes, contenant un composé pharmaceutiquement actif.

18. Capsule selon la revendication 17, dans laquelle la matière active est une matière solide particulaire.

19. Capsule selon la revendication 18, dans laquelle le composé actif est présent sous la forme d'une forme de dosage modifiée ayant un profil de libération autre que celui à impulsion unique.

20. Capsule selon l'une quelconque des revendications précédentes, ayant une surface externe dotée d'un revêtement gastro-résistant.

21. Procédé de préparation d'une capsule à libération contrôlée par impulsions, comprenant l'étape consistant à insérer dans l'orifice d'une capsule ayant une surface imperméable à l'eau un bouchon précomprimé hydrosoluble ou êrodable dans l'eau composé d'une matière pharmaceutiquement acceptable qui se dissout ou s'érode dans l'eau au bout d'un certain temps dans le corps humain, le bouchon et la capsule définissant ensemble une cavité contenant le contenu de la capsule, dans lequel l'aire de la matière hydrosoluble ou érodable dans l'eau est suffisante pour permettre la pénétration rapide d'eau dans la cavité lorsque la matière s'est dissoute ou érodée.
